# EUROPEAN PATENT APPLICATION

(11) **EP 2 871 064 A1**
(43) Date of publication of application: **13.05.2015**
(21) Application number: 14003769.8
(22) Date of filing: 10.11.2014
(51) Int. Cl.: B43L 9/00, A61B 5/00

(54) **Device for monitoring skin lesions and neoformations**

(30) Priority: 11.11.2013 IT MI20130391 U
(71) Applicant: Viappiani, Giorgio, 20096 Pioltello (Milano) (IT)
(72) Inventor: Viappiani, Giorgio, 20096 Pioltello (Milano) (IT)
(74) Representative: Forattini, Amelia

(57) **Abstract**

A device (1) for monitoring skin lesions and neoformations, characterized in that it includes a sheet member (2) and is provided with a series of calibrated circles (3), each of which is identified with its own diameter; the sheet member being superimposable on the skin of a patient so as to record the dimensions of a skin lesion that corresponds to a given calibrated circle.

## Description

The present invention relates to a device for monitoring skin lesions and neoformations.

As is known, the term nevus, more commonly mole, designates a benign tumor of the skin, which has the appearance of a persistent blemish on the skin, and in general any localized and persistent color or shape alteration that is present on the surface of the skin.

In scientific language, the term nevus is followed by an adjective that defines it better and is used to indicate specific skin lesions or neoformations.

Many types of nevi are known and the term nevus is used improperly to designate the most disparate skin lesions, from angiomas to fibromas, from melanoses to keratoses.

Among the most important skin lesions to be monitored one must consider melanoma, which can arise de novo or from an existing nevus.

Changes in symmetry, edges, color, size and extension of an existing nevus lead to suspicion of neoplastic growth.

The size of the nevus and its evolution are very important parameters to verify; however, the rapid growth of a nevus occurs over weeks or months and therefore it can be difficult to appreciate its variation.

Another problem is that once the size variation of the nevus has been ascertained in some manner, it is however impossible for the patient to indicate to the physician the exact extent of this variation and the elapsed time.

The aim of the present invention is to provide a device for monitoring skin lesions and neoformations that allows to establish with certainty whether a lesion has changed size.

Within the scope of this aim, an object of the invention is to provide a device that allows to rapidly and easily record the data acquired on a lesion.

Another object is to provide a device that can be used by the patient, without the intervention of specialists.

Another object of the present invention is to provide a device that can be provided easily by using commonly commercially available elements and materials and is furthermore economic so as to facilitate its diffusion.

This aim, these objects and others that will become better apparent hereinafter are achieved by a device for monitoring skin lesions and neoformations, characterized in that it comprises a sheet member and is provided with a set of calibrated circles, each of which is identified with its own diameter; said sheet member being superimposable on the skin of a patient so as to record the dimensions of a skin lesion that corresponds to a given calibrated circle.

Further characteristics and advantages will become better apparent from the description of the structure according to the invention, illustrated by way of non-limiting example in the accompanying drawings, wherein:

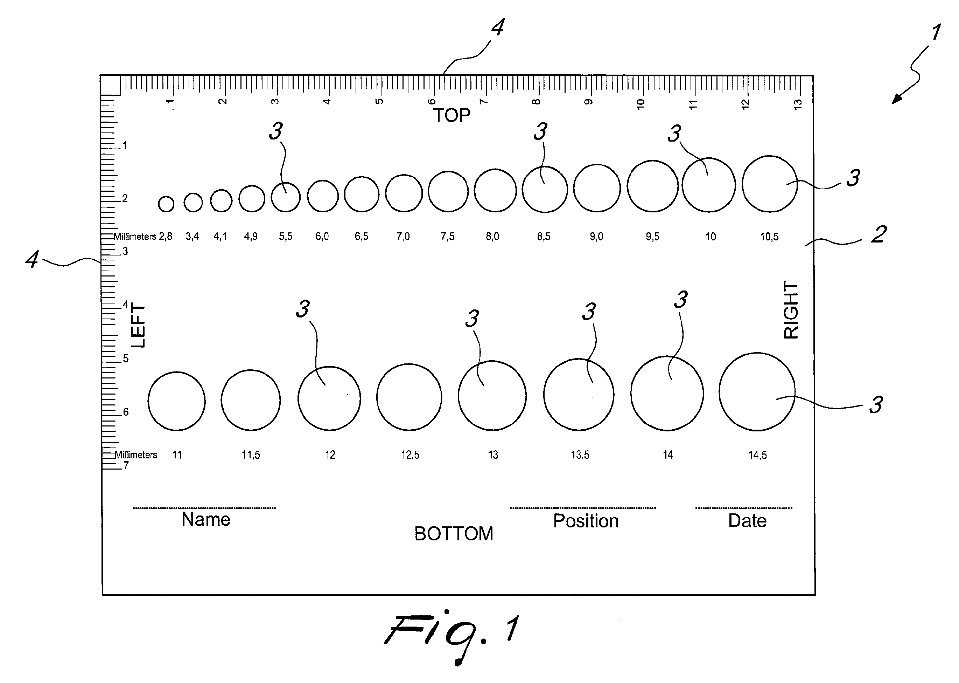

Figure 1 is a plan view of the device according to the present invention;

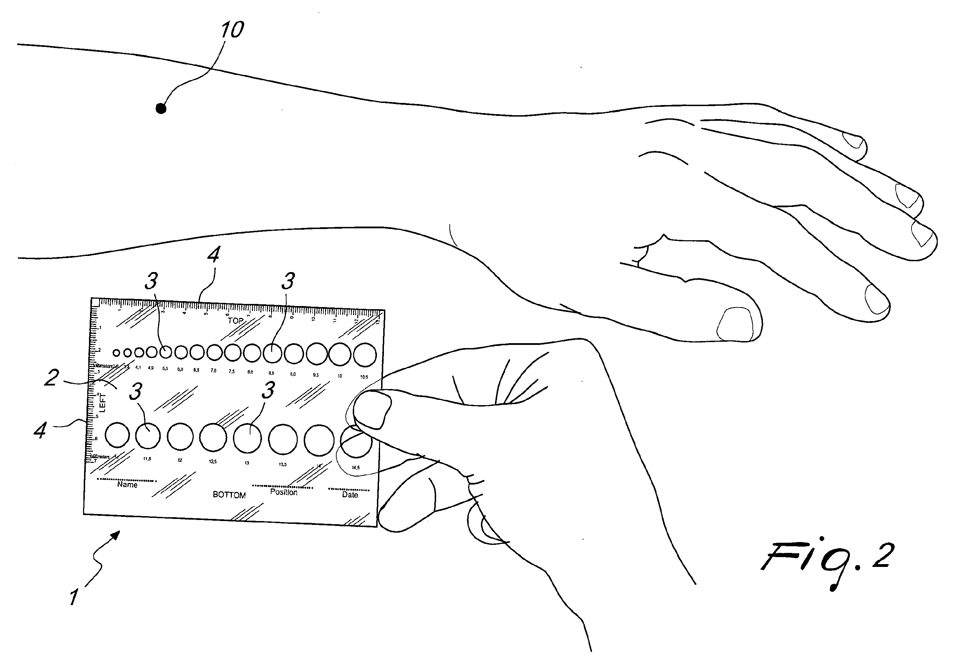

Figure 2 shows the use of the device according to the present invention;

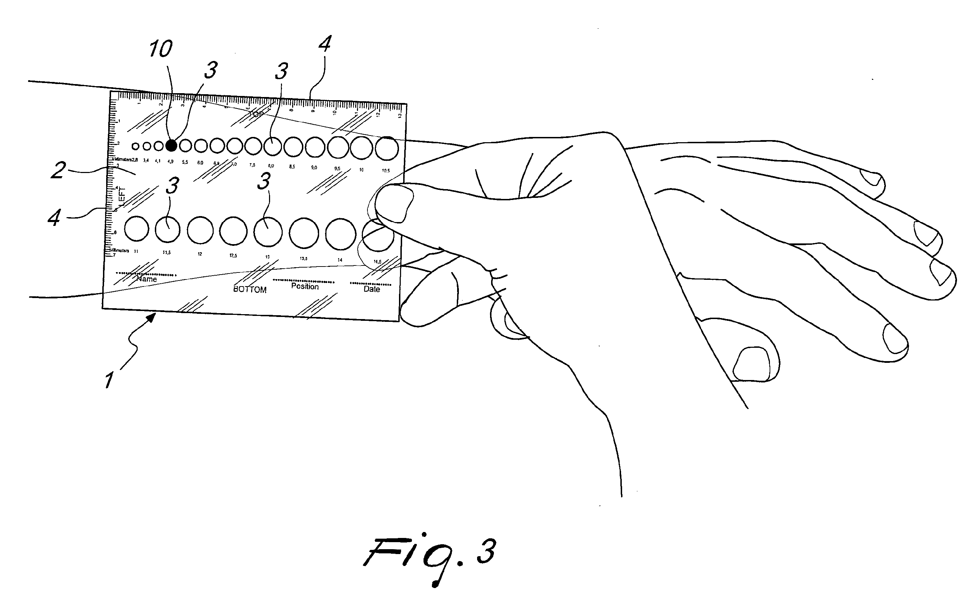

Figure 3 shows the device applied to the skin of the patient in order to detect the lesion.

With reference to the cited figures, the device for monitoring skin lesions and neoformations according to the invention, generally designated by the reference numeral 1, includes a sheet member 2 that is preferably made of flexible material, such as a sheet of transparent plastic.

The sheet member 2 has a series of calibrated circles 3, each of which is identified by means of its own diameter, preferably expressed in millimeters, or another suitable unit of measure.

The sheet member 2 also, preferably, has one or more graduated scales, designated by the reference numeral 4, arranged on one or more sides of the sheet member 2.

In order to facilitate placement and optional photographic detection, the sheet member 2 is provided with "up", "down", "left", "right" indications on its four sides.

The sheet member 2 is also provided with adequate spaces for applying various indications, such as the location of the monitored lesion, the detection date and the name of the patient.

Advantageously, the device 1 has an auxiliary sheet, not shown in the figures, which shows the image of the human body so as to allow the user to indicate on the image itself the exact location of the lesion and the data related thereto.

The sheet member 2 and/or the auxiliary sheet can bear the printed indication of the name of the physician who performed the detection, acting as a visiting card and as a reminder.

The sheet member 2 and/or the auxiliary sheet can have an advertising space.

The device according to the present invention is used as follows.

The device is held with one hand and is superimposed on the lesion 10 to be monitored, identifying the calibrated circle 3 that corresponds to the dimensions of the lesion 10.

The correct calibrated circle must be identified by checking also the directly adjacent smaller and larger circles.

The size of the calibrated circle 3 thus identified is noted and so is its location on the body as well as date of the detection.

Optionally, a photograph of the lesion is taken, with the device superimposed thereon, so as to document the location of the lesion and the corresponding calibrated circle, for subsequent comparison, with the detection performed at a later time.

This can be done advantageously by using applications for smartphones, dedicated or otherwise, programs for personal computers, optionally via the Internet, and also by using systems for digital storage of photographs and data.

The applications (apps) or the Internet programs allow the user to photograph, store and catalog on a memory/database the various photographs, taken at different times, of each lesion being monitored and the corresponding data related to it, so as to obtain a personal archive of the evolution of the lesion.

This measurement is performed multiple times, at preset intervals, so that any dimensional variation of the nevus is detected immediately and unambiguously, since the nevus is no longer comprised within the calibrated circle of the measurement performed previously.

The present device allows to immediately detect that a size variation of the nevus has occurred and, by virtue of the indications duly recorded on the sheet member 2, or on the auxiliary sheet, allows to provide precise size and time data of this variation, which are useful for diagnosis of the case.

According to a practical embodiment of the invention, shown in the figures, the sheet member is constituted by a sheet made of transparent material and the calibrated circles are constituted by holes.

According to a further embodiment, the calibrated circles are constituted by circles that are printed on the transparent sheet without perforating the sheet itself.

According to a further embodiment, the sheet member is constituted by a sheet made of opaque material and the calibrated circles are constituted by holes or transparent regions.

In practice it has been found that the invention achieves the intended aim and objects, providing an aid to compare any dimensional variation over time of a skin lesion, allowing a reliable monitoring and verification thereof.

The device according to the present invention solves the problem of checking any size and shape variations of moles, in a fully objective manner, without depending on visual memory and on inaccurate observations performed without instruments.

The materials and the dimensions may of course vary according to the requirements and the state of the art.

## Claims

1. A device for monitoring skin lesions and neoformations, **characterized in that** it comprises a sheet member and is provided with a set of calibrated circles, each of which is identified with its own diameter; said sheet member being superimposable on the skin of a patient so as to record the dimensions of a skin lesion that corresponds to a given calibrated circle.

2. The device according to claim 1, **characterized in that** said sheet member is made of flexible, transparent material.

3. The device according to claim 1 or 2, **characterized in that** said sheet member has one or more graduated scales arranged on one or more sides of said sheet member.

4. The device according to one or more of the preceding claims, **characterized in that** said sheet member bears the indications "up", "down", "left" and "right" on its sides.

5. The device according to one or more of the preceding claims, **characterized in that** said sheet member is provided with spaces to apply various indications, such as the location of the monitored lesion, the detection date and the name of the patient.

6. The device according to one or more of the preceding claims, **characterized in that** it comprises one or more applications for smartphones, programs for personal computers, optionally via the Internet, and systems for digital storage of photographs and data; said applications and/or programs allowing the user to photograph, store and catalog in a memory/database the various photographs, taken at different times, of each lesion being monitored and the corresponding data thereof, so as to obtain a personal archive of the evolution of the lesion.

7. The device according to one or more of the preceding claims, **characterized in that** it comprises an auxiliary sheet, which shows the image of the human body so as to allow the user to indicate on said image the exact position of the lesion and the data related thereto.

8. The device according to one or more of the preceding claims, **characterized in that** said sheet member and/or said auxiliary sheet bear the printed indication of the name of the physician who performed the detection, acting as a visiting card and as a reminder.

9. The device according to one or more of the preceding claims, **characterized in that** said sheet member and/or said auxiliary sheet have at least one advertising space.
